# EUROPÄISCHE PATENTANMELDUNG

(11) **EP 1 186 279 A1**
(43) Veröffentlichungstag der Anmeldung: **13.03.2002**
(21) Anmeldenummer: 00119551.0
(22) Anmeldetag: 07.09.2000
(51) Int. Cl.: A61F 5/01

(54) **Gelenkorthese mit bewegungsinduzierter Entriegelung**

(71) Anmelder: Ferdinand Hauber GmbH & Co. KG, 72622 Nürtingen (DE)
(72) Erfinder: Wirz, Peter, Dr., 3508 Arni (CH)
(74) Vertreter: Hellwig, Tillmann, Dr.

(57) **Zusammenfassung**

Es wird eine Gelenkorthese, insbesondere für eine Kniegelenk, vorgeschlagen, die in Abhängigkeit der Belastungs des Beins und der Stellung des Knies verriegelt oder entriegelt wird. Mit der erfindungsgemäßen Orthese wird das Gangbild von Patienten mit geschwächter Oberschenkelmuskulatur verbessert und die untere Wirbelsäule entlastet.

## Beschreibung

### Beschreibung

Die Erfindung betrifft eine Gelenkorthese zur Unterstützung eines Gelenks, insbesondere eines Kniegelenks, mit einem ersten Gelenkglied, mit einem zweiten Gelenkglied, mit einem Zahnrichtgesperre, wobei das erste Gelenkglied und das zweite Gelenkglied mit einem Gelenkbolzen drehbar miteinander verbunden sind, wobei das erste Gelenkglied eine Verzahnung aufweist und wobei an dem zweiten Gelenkglied eine mit der Verzahnung zusammenwirkende Zahnklinke drehbar angeordnet ist.

Diese aus der US-PS 2,943,622 bekannte Gelenkorthese dient dazu ein menschliches Kniegelenk zu unterstützen. Dies u. a. bei Patienten, die aufgrund einer temporären oder dauernden Muskelschwäche im Oberschenkel ohne Orthese nicht laufen und nicht sicher stehen können. Der Drehbereich der Gelenkorthese wird durch zwei Endanschläge festgelegt. Dadurch ist sichergestellt, dass bei gestrecktem Bein das Knie nicht überstreckt wird und der Patient sicher stehen kann. Das Zahnrichtgesperre ist in dieser Stellung verriegelt.

Ein zweiter Anschlag der aus der US-PS 2,943,622 bekannten Gelenkorthese verhindert, dass das Kniegelenk des Patienten zu weit abgewinkelt wird. Durch das Zahnrichtgesperre ist die Gelenkorthese auch in Zwischenstellungen verriegelt, so dass das Bein des Patienten beim Aufstehen nicht ungewollterweise im Kniegelenk abwinkelt wird und der Patient dadurch möglicherweise das Gleichgewicht verliert. Entriegelt wird die Orthese durch eine Auslöseeinrichtung, welche im Fersenbereich des Patienten in einem Schuh angeordnet ist. Sobald die Ferse entlastet wird entriegelt das Zahnrichtgesperre und erlaubt somit ein Abknicken des Knies, wie es beim Hinsitzen der Fall ist.

Bei der bekannten Orthese läuft der Patient, indem er zunächst das durch die Orthese nach dem Stand der Technik versteifte Bein mit einer entsprechenden Hüftbewegung anhebt und anschließend mit dem Bein eine halbkreisförmige Bewegung nach vorne und zur Seite ausführt. Dieser unnatürliche Bewegungsablauf belastet vor allem die untere Wirbelsäule stark und hat ein unnatürliches Gangbild zur Folge.

Der Erfindung liegt die Aufgabe zu Grunde, die bekannte Gelenkorthese so weiterzubilden, dass der Patient mit Hilfe der Gelenkorthese beim Laufen unterstützt wird und das Laufbild des Patienten so weit wie möglich einem natürlichen Bewegungsablauf angenähert wird.

Diese Aufgabe wird erfindungsgemäß gelöst durch eine Gelenkorthese zur Unterstützung eines Gelenks, insbesondere eines Kniegelenks, mit einem ersten Gelenkglied, mit einem zweiten Gelenkglied, mit einem Zahnrichtgesperre, wobei das erste Gelenkglied und das zweite Gelenkglied mit einem Gelenkbolzen drehbar miteinander verbunden sind, wobei das erste Gelenkglied ein Sperrstück mit einer Verzahnung aufweist, wobei an dem zweiten Gelenkglied eine mit der Verzahnung zusammenwirkende Zahnklinke drehbar angeordnet ist und wobei das Zahnrichtgesperre beim Erreichen eines ersten Drehwinkelanschlags entriegelt wird.

Durch das Entriegeln des Zahnrichtgesperres bei durchgestrecktem Bein besteht die Möglichkeit für den Patienten, durch Drehen des Oberschenkels im Hüftgelenk einen Schritt mit dem von einer erfindungsgemäßen Gelenkorthese gestützen Bein auszuführen, welcher weitgehend einem natürlichen Bewegungsablauf ähnelt, ohne dass die Oberschenkelmuskulatur des mit der Gelenkorthese versehenen Beins eine Kraft auf den Unterschenkel ausüben muss. Der Unterschenkel folgt wegen der Kopplung über das Kniegelenk und die Gelenkorthese der Bewegung des Oberschenkels und führt aufgrund seiner Massenträgheit eine dem natürlichen Bewegungsablauf sehr ähnliche Bewegung aus. In dieser Schwungphase ist das Bein entlastet und die Verriegelung geöffnet.

Damit ist für die mit der erfindungsgemäßen Gelenkorthese versehenen Patienten ein Gehen ohne unnatürliches und die untere Wirbelsäule stark belastendes Anheben der Hüfte mit gleichzeitigem kreisförmigen Ausweichen des durch eine Orthese (US-PS 2,943,622) versteiften Kniegelenks möglich. Neben den biomechanischen Vorteilen, welche durch den Einsatz der erfindungsgemäßen Gelenkorthese erzielt werden, stellt sich bei den Patienten ein verbessertes Wohlbefinden und ein erhöhtes Selbstwertgefühl aufgrund des verbesserten Gangbilds ein.

Bei einer Variante der Erfindung ist vorgesehen, dass das erste Gelenkglied einen ersten Nocken aufweist, der beim Erreichen des ersten Drehwinkelanschlags die Zahnklinke aus der Verzahnung hebt, so dass ein Überstrecken des Kniegelenks wegen des ersten Drehwinkelanschlags nach wie vor unmöglich ist, andererseits das Kniegelenk abknicken kann, wenn der Patient den Oberschenkel beim Laufen anhebt, um einen Schritt zu setzen.

Eine Ausgestaltung der Erfindung sieht vor, dass der Eingriffswinkel α der Verzahnung in Belastungsrichtung negativ ist und vorzugsweise -10° bis -30°, besonders bevorzugt -20° beträgt, so dass mit zunehmender Belastung des Zahnrichtgesperres die Zahnklinke immer fester in die Verzahnung hineingedreht wird und somit ein unbeabsichtigtes Lösen des Zahnrichtgesperres unter Belastung unmöglich ist.

In weiterer Ergänzung der Erfindung ist vorgesehen, dass das zweite Gelenkglied die Verzahnung beidseitig umgibt und dass der Gelenkbolzen zweifach in dem zweiten Gelenkstück gelagert ist, so dass sich eine symmetrische Belastung der Gelenkorthese ergibt und somit deren Belastbarkeit und Lebensdauer erhöht wird. Außerdem ist bei dieser Ausgestaltung die Verzahnung durch das zweite Gelenkglied weitestgehend abgedeckt, so dass die Funktionsweise der Verzahnung nicht durch eine Hose oder dergleichen beeinträchtigt werden kann. Im Gegenzug kann die Verzahnung bei dieser Ausführungsform keine Verletzungen des Patienten oder Beschädigungen, beispielsweise einer Hose, verursachen.

Um die Lebensdauer und die Belastbarkeit der Gelenkorthese weiter zu steigern, ist vorgesehen, zwischen Gelenkbolzen sowie erstem und zweiten Gelenkglied eine Büchse, insbesondere aus Bronze mit Teflonbeschichtung, vorzusehen.

Die Belastbarkeit und Lebensdauer der erfindungsgemäßen Gelenkorthese wird weiter gesteigert, wenn zu beiden Seiten des zu unterstützenden Gelenks, wie beispielsweise dem Knie, je ein Gelenk mit einem ersten Gelenkglied, einem zweiten Gelenkglied und einem Zahnrichtgesperre angeordnet ist, wobei die Drehachsen der Gelenke koaxial verlaufen.

Um zu verhindern, dass das zu unterstützende Gelenk zu stark abgewinkelt wird, weist bei einer anderen Ausgestaltung der erfindungsgemäßen Gelenkorthese das erste Gelenkglied einen zweiten Nocken auf, der beim Erreichen eines zweiten Drehwinkelanschlags die Zahnklinke in die Verzahnung einrastet. Dadurch dass das von der Gelenkorthese übertragende Drehmoment, welches sich aus der Gewichtskraft des Patienten multipliziert mit der wirksamen Hebellänge ergibt, nicht ausschließlich vom zweiten Drehwinkelanschlag übertragen werden muss, sondern auch von dem Zahnrichtgesperre übertragen wird, wird die Belastbarkeit der erfindungsgemäßen Gelenkorthese gesteigert.

Weitere erfindungsgemäße Ausgestaltungen sehen vor, dass das Zahnrichtgesperre von einer Auslöseeinrichtung entriegelbar ist, so dass das Verriegeln oder Entriegeln des Zahnrichtgesperres durch die von der Bewegung des Patienten gesteuerten Auslöseeinrichtung erfolgt. Dabei ist es besonders vorteilhaft, wenn die Zahnklinke federbelastet in die Verzahnung bewegt wird und von der Auslöseeinrichtung aus der Verzahnung gedreht werden kann, so dass bei einer Funktionsstörung der Auslöseeinrichtung oder einer fehlerhaften Montage derselben die erfindungsgemäße Gelenkorthese in gleicher Weise wie die aus dem Stand der Technik bekannte Gelenkorthese einsetzbar bleibt. D. h. die Gelenkorthese verriegelt und der Patient kann nicht stürzen.

Eine Ergänzung der Erfindung sieht vor, dass die Auslöseeinrichtung in oder an einem Schuh angeordnet ist, dass die Auslöseeinrichtung eine drehbar in einer Wippenführung gelagerte Wippe aufweist, dass sich an der Wippenführung und an dem zweiten Gelenkglied eine Bowdenzughülle mindestens mittelbar abstützt, und dass Wippe und Zahnklinke durch die Seele des Bowdenzugs mindestens mittelbar gekoppelt sind, so dass durch Entlasten des Fußes die Zahnklinke von der Auslöseeinrichtung aus der Verzahnung gedreht wird und somit das Kniegelenk abgewinkelt werden kann. Das Entlasten des Fußes findet genau dann statt, wenn der Patient den Oberschenkel anhebt, um einen Schritt nach vorne zu setzen. Alternativ kann, wie bereits erwähnt, das Zahnrichtgesperre auch ausgelöst werden, wenn der Patient das Bein ganz durchstreckt und der erste Nocken die Zahnklinke aus der Verzahnung hebt. Da mit dem Abwinkeln des Kniegelenks auch eine Entlastung des Fußes, insbesondere dessen Ferse, einhergeht, sorgt die Auslöseeinrichtung dafür, dass während des gesamten Bewegungsablaufs bis zum Aufsetzen der Ferse das Zahnrichtgesperre entriegelt bleibt und sich das gewünschte schöne Gangbild einstellt.

Weitere Ausgestaltungen der Erfindung sehen vor, dass an der Wippenführung eine Drehwinkelbegrenzung für die Wippe vorhanden ist und/oder dass zwischen Wippenführung und Wippe eine Vorfußwippe angeordnet ist, dass die Vorfußwippe koaxial zum Drehpunkt der Wippe gelagert ist, und dass die Wippenführung und die Fußwippe über den Drehpunkt der Wippe hinaus verlängert sind, so dass auch durch ein Betätigen der Vorfußwippe durch den Ballen des Fußes die Auslöseeinrichtung betätigt und das Zahnrichtgesperre entriegelt werden kann. Dadurch ist eine weitere Erleichterung des Patienten gegeben, da er mit Hilfe seines Fußballens die Gelenkorthese zusätzlich entriegeln kann. Die Befestigung des Bowdenzugs befindet sich dabei auf der Vorfußwippe.

Um stets einen definierten Zustand der Gelenkorthese zu haben, ist vorgesehen, dass ein Federelement die Wippe so zur Wippenführung und/oder zur Vorfußwippe verdreht, dass die Zahnklinke durch das Federelement aus der Verzahnung gedreht wird. Das heißt, nur wenn die Ferse des Patienten durch dessen Gewicht belastet wird oder der Patient aktiv die Fersenwippe oder Vorfußwippe betätigt, wird das Zahnrichtgesperre entriegelt.

Zur individuellen Einstellung und Adaption an den Patienten ist zwischen Auslöseeinrichtung und Gelenk eine Einstelleinrichtung, insbesondere eine Stellschraube, vorgesehen. Des Weiteren ist vorgesehen, dass Zwischenauslöseeinrichtung und Gelenk eine Kupplung, insbesondere bestehend aus einem ersten, mit dem zweiten Gelenkglied mindestens mittelbar verbundenen ersten Kupplungsstück und einem mit der Auslöseeinrichtung mindestens mittelbar verbundenen zweiten Kupplungsstück, die miteinander formschlüssig verbindbar sind, so dass Gelenkorthese und Auslöseeinrichtung auch vom Patienten auf einfache Weise voneinander getrennt werden können. Dadurch vereinfacht sich das An- und Ablegen der erfindungsgemäßen Gelenkorthese und der Patient kann, wenn er will, verschiedene Schuhe in Verbindung mit der erfindungsgemäßen Gelenkorthese benützen. Um die Kupplung zu sichern kasnn diese in einem Gehäuse untergebracht werden. Zur Vermeidung von Interferenzen zwischen den beidseitig eines zu unterstützenden Gelenks angebrachten Gelenken einer Gelenkorthese können herkömmliche Stellschrauben odgl., wie sie bei Bowdenzügen bekannt sind, eingesetzt werden.

Bei einer anderen Variante ist vorgesehen, dass dass die Zahnklinke von Hand in die Verzahnung oder aus der Verzahnung gedreht werden und in beiden Positionen sowie in eine Neurtalstellung Zwischenpositionen feststellbar ist, so dass die erfindungsgemäße Gelenkorthese auch wie eine aus dem Stand der Technik bekannte Gelenkorthese einsetzbar ist.

Weitere Vorteile und vorteilhafte Ausgestaltungen der Erfindung sind der nachfolgenden Zeichnung, deren Beschreibung und den Patentansprüchen entnehmbar. Es zeigen:
- Fig. 1: eine erfindungsgemäße Orthese mit einer Auslöseeinrichtung,
- Fig. 2-6: verschiedene Positionen des Zahnrichtgesperres,
- Fig. 7: ein zweites Ausführungsbeispiel einer erfindungsgemäßen Auslöseeinrichtung und
- Fig. 8: ein Ausführungsbeispiel einer erfindungsgemäßen Kupplung.

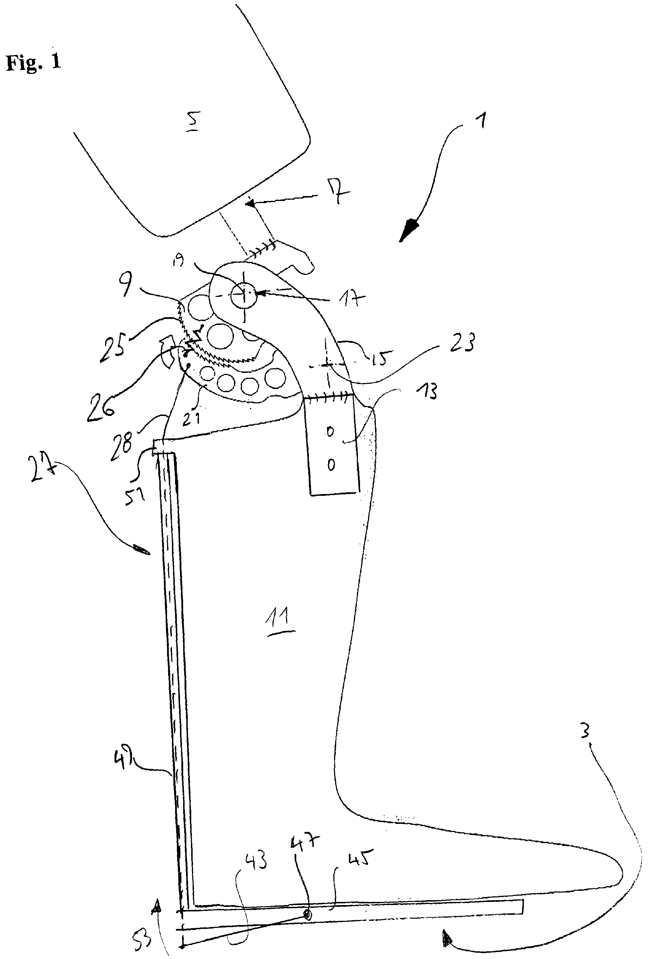

In Figur 1 ist ein Ausführungsbeispiel einer erfindungsgemäßen Gelenkorthese 1, die der Unterstützung eines nicht dargestellten Kniegelenks dient, und eine Auslöseeinrichtung 3 schematisiert dargestellt. Der Einsatz der erfindungsgemäßen Gelenkorthese 1 ist nicht auf Kniegelenke beschränkt.

Ein Oberteil 5, welches am nicht dargestellten Oberschenkel des Patienten befestigt wird, ist über ein oberes Anschlussteil 7 mit einem ersten Gelenkglied 9 verbunden. Oberes Anschlussteil 7 und erstes Gelenkglied 9 können beispielsweise durch Schweißen miteinander verbunden sein, während das obere Anschlussteil 7 und das Oberteil 5 verschraubt, vernietet oder verklebt sein können.

Ein Unterteil 11 ist über ein unteres Anschlussteil 13 mit einem zweiten Gelenkglied 15 verbunden. Das Unterteil umfasst bei dem Ausführungsbeispiel nach Fig. 1 Unterschenkel und Fuß des nicht dargestellten Patienten. Die erfindungsgemäße Gelenkorthese kann im Bereich des Knöchels des Patienten ein weiteres, in Fig. 1 nicht dargestelltes Gelenk aufweisen, so dass der Patient den Fuß relativ zum Unterschenkel abwinkeln kann.

Das erste Gelenkglied 9 ist mittels eines Gelenkbolzens 17 drehbar im zweiten Gelenkglied 15 gelagert. Der Gelenkbolzen 17 legt somit den Drehpunkt der Gelenkorthese 1, nachfolgend als erster Drehpunkt bezeichnet, fest. Um den hohen Belastungen besser standhalten zu können, kann zwischen Gelenkbolzen 17 und erstem Gelenkglied 9 sowie zweitem Gelenkglied 15 jeweils eine teflonbeschichtete Bronzebuchse vorgesehen sein. Diese Bronzebuchse ist in Figur 1 nicht dargestellt.

Am zweiten Gelenkglied 15 ist eine Zahnklinke 21 angeordnet, die um einen zweiten Drehpunkt 23 drehbar zum zweiten Gelenkglied 15 gelagert ist. Die Lagerung der Zahnklinke 21 im zweiten Gelenkglied 15 kann in gleicher Weise wie die Lagerung des ersten Gelenkglieds 9 erfolgen. Die Zahnklinke 21 kann in eine Verzahnung 25 des ersten Gelenkglieds 9 einrasten und somit ein weiteres Abwinkeln von Oberteil 5 relativ zum Unterteil 11 blockieren. Oberes Anschlussteil 7, erstes Gelenkglied 9, unteres Anschlussteil 13, zweites Gelenkglied 15 und/oder Zahnklinke 21 können aus härtbarem, bervorzugt nichtrostendem Stahl angefertigt werden.

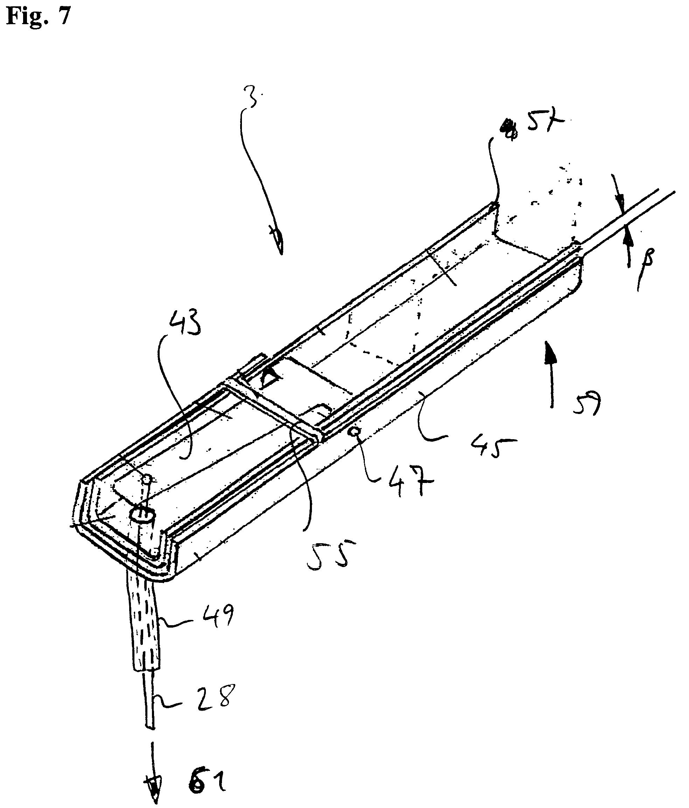

Zwischen Zahnklinke 21 und erstem Gelenkglied 9 ist eine Zugfeder 26 angeordnet, die die Zahnklinke 21 in die Verzahnung 25 dreht. Betätigt wird die Zahnklinke 21 unter anderem von einem Bowdenzug 27, der an seinem anderen Ende mit der Auslöseeinrichtung 3 verbunden ist. Das in Fig. vereinfacht dargestellte erste Ausführungsbeispiel einer Auslöseeinrichtung 3 wird weiter unten vor Fig. 7 beschrieben. Alternativ kann die Zahnklinke 21 auch durch ein mit einer Auslöseeinrichtung verbundenes Gestänge betätigt werden. In diesem Fall ist die Zugfeder 26 entbehrlich.
Nicht dargestellt ist die optional vorgesehene manuelle Betätigung des Zahnrichtgesperres 31, welche ebenfalls über einen Nocken erfolgen kann.

Das zweite Gelenkglied 15 ist in der Seitenansicht U-förmig ausgebildet und umfasst das erste Gelenkglied 9 von beiden Seiten, so dass der Gelenkbolzen 17 an beiden Enden im zweiten Gelenkglied 15 gelagert ist und in der Mitte vom ersten Gelenkglied 9 aufgenommen wird. Dadurch wird die Belastbarkeit der Gelenkorthese 1 erhöht.

Im Folgenden werden für gleiche Bauteile in den verschiedenen Figuren die gleichen Bezugszeichen verwandt und nicht nochmals erläutert. Es gilt das anhand einer Figur Erläuterte für die anderen Figuren, in denen gleiche Bezugszeichen auftreten, entsprechend.

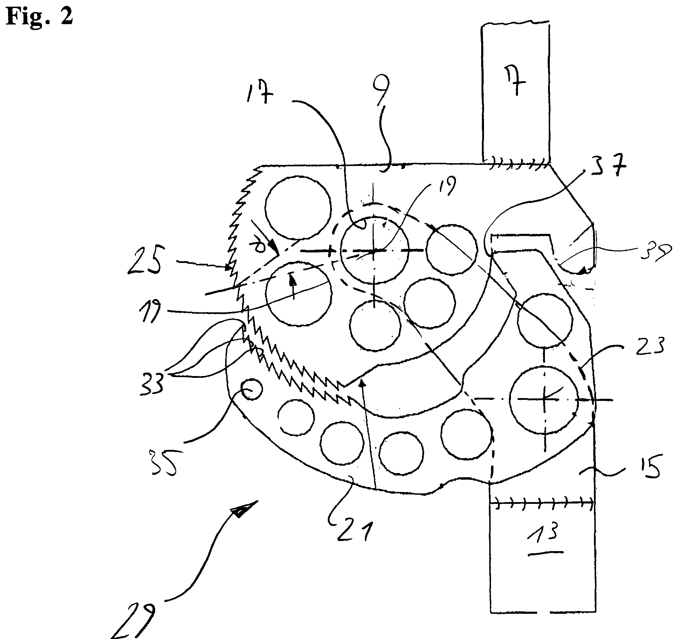

In Figur 2 ist ein erfindungsgemäßes Gelenk, welches im Wesentlichen aus erstem Gelenkglied 9, zweitem Gelenkglied 15 und Zahnklinke 21 besteht, vergrößert dargestellt. Dabei wurde zur Verbesserung der Übersichtlichkeit die dem Betrachter zugewandte Seite des U-förmigen zweiten Gelenkglieds 15 weggelassen. Der hinter der Zahnklinke 21 und dem ersten Gelenkglied 9 liegende Teil des zweiten Gelenkglieds 15 ist in Figur 2 gestrichelt dargestellt. In der Position, die oberes Anschlussteil 7 und unteres Anschlussteil 13 in Fig. 2 einnehmen, ist das Bein eines mit einer erfindungsgemäßen Gelenkorthese versehenen Patienten gerade durchgestreckt. Das aus Verzahnung 25 und Zahnklinke 21 bestehende Zahnrichtgesperre 31 ist geöffnet, da die Verzahnung 25 und die Zähne 33 der Zahnklinke 21 nicht im Eingriff sind. Die Öffnung des Zahnrichtgesperres kann entgegen der Federkraft der in Figur 1 dargestellten Zugfeder 26, welche an erstem Gelenkglied 9 und Zahnklinke 21 befestigt ist, durch eine nicht dargestellte Seele des Bowdenzugs 27, welche an einer Aufnahmebohrung 35 der Zahnklinke eingehängt ist, bewirkt werden. Die Seele des Bowdenzug 27 kann natürlich auch auf andere Weise an der Zahnklinke 21 befestigt werden. Damit die Zahnklinke 21 nicht zu weit öffnet, ist am zweiten Gelenkglied 15 eine Nase 37 vorgesehen, welche sich an dem ersten Gelenkglied 9 abstützt.

Damit die Verriegelung zwischen erstem Gelenkglied 9 und zweitem Gelenkglied 15 sich nicht aufgrund einer hohen Belastung öffnet, sind die Zähne der Verzahnung 25 asymmetrisch ausgeführt. Zieht man vom ersten Drehpunkt 19 zu der Spitze eines Zahns der Verzahnung 25 eine Verbindungslinie und bezeichnet den zwischen der Zahnflanke des Zahns in Sperrrichtung und dieser Verbindungslinie eingeschlossenen Winkel als Eingriffswinkel α, so ist der Eingriffswinkel α bei dem Ausführungsbeispiel gemäß Figur 2 negativ. Es hat sich als vorteilhaft erwiesen, den Eingriffswinkel α etwa auf -20° festzulegen. Die Zähne 33 der Zahnklinke 21 sind entsprechend den Zähnen der Verzahnung 25 geformt. Der Eingriffswinkel der anderen Zahnflanke der Zähne 25 ist größer als +20°, bevorzugt 50° - 70°. Dieser Eingriffswinkel ist aus Gründen der Übersichtlichkeit in Figur 2 nicht eingetragen.

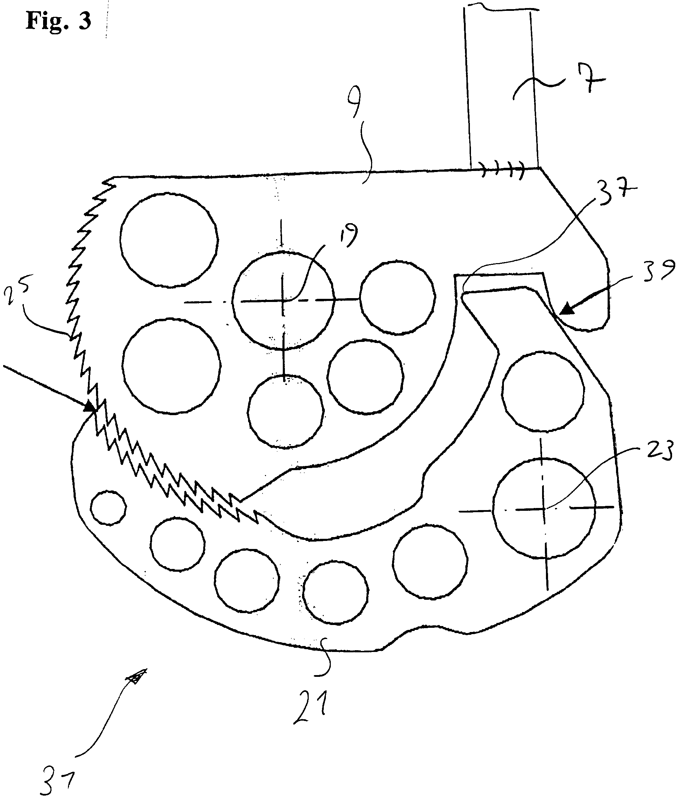

Wenn das Gelenk 29 noch etwas weiter als in Fig, 2 dargestellt geöffnet wird, entsprechend einem durchgestreckten Kniegelenk des Patienten, drückt ein erster Nocken 39 des ersten Gelenkglieds 9 die Zahnklinke 21 aus der Verzahnung 25 des ersten Gelenkglieds 9. Der erste Nocken 39 drückt dabei auf die Rückseite der Nase 37 der Zahnklinke 21. Diese Situation ist in Figur 3 dargestellt. Auf die Darstellung des zweiten Gelenkglieds 15 wurde hierbei verzichtet. Diese Funktion ermöglicht es einem Patienten, die Gelenkorthese zu entriegeln, indem er sein Bein etwas überstreckt.

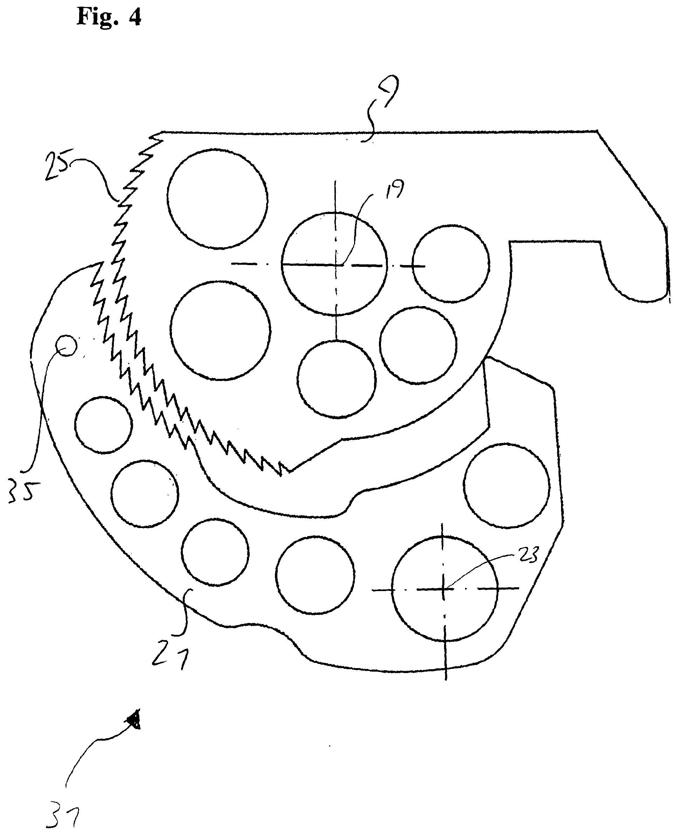

In Figur 4 ist das Gelenk 29 leicht abgewinkelt und mit geöffnetem Zahnrichtgesperre 31 dargestellt.

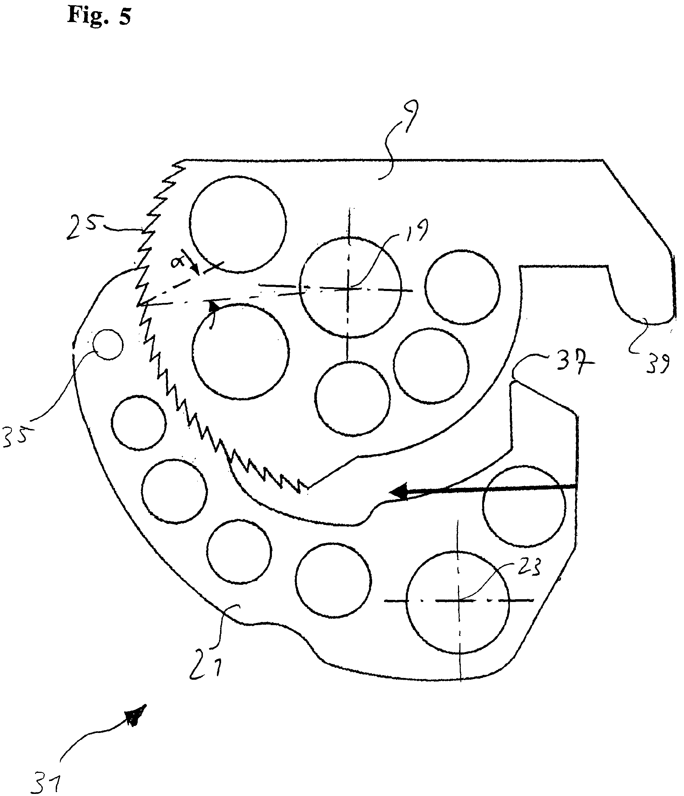

In Figur 5 ist das Gelenk 29 mit eingerasteter Zahnklinke 21 dargestellt. Dabei ist deutlich zu erkennen, dass alle Zähne 33 der Zahnklinke 21, die der Übersichtlichkeit wegen in Figur 5 nicht einzeln mit Bezugszeichen versehen wurden, mit der Verzahnung 25 im Eingriff sind. Dadurch ist es möglich, zwischen erstem Gelenkglied 9 und dem in Figur 5 nicht dargestellten zweiten Gelenkglied 15 sehr hohe Drehmomente zu übertragen. Aus Figur 5 wird auch die Wirkung des negativen Eingriffwinkels α ersichtlich, welche darin besteht, dass mit zunehmender Belastung der Gelenkorthese 1 entgegen dem Uhrzeigersinn die Zahnklinke 21 immer fester in die Verzahnung 25 gezogen wird. In dieser Belastungsrichtung muß das Körpergewicht des Patienten von der Gelenkorthese übertragen werden, wenn die Muskulatur des Patienten dazu nicht in der Lage ist. Die andere Bewegungsrichtung stellt sich beim Aufstehen des Patienten ein. Wird das Gelenk aktiv vom Patienten gestreckt, so löst sich das Zahnrichtgesperre 31.

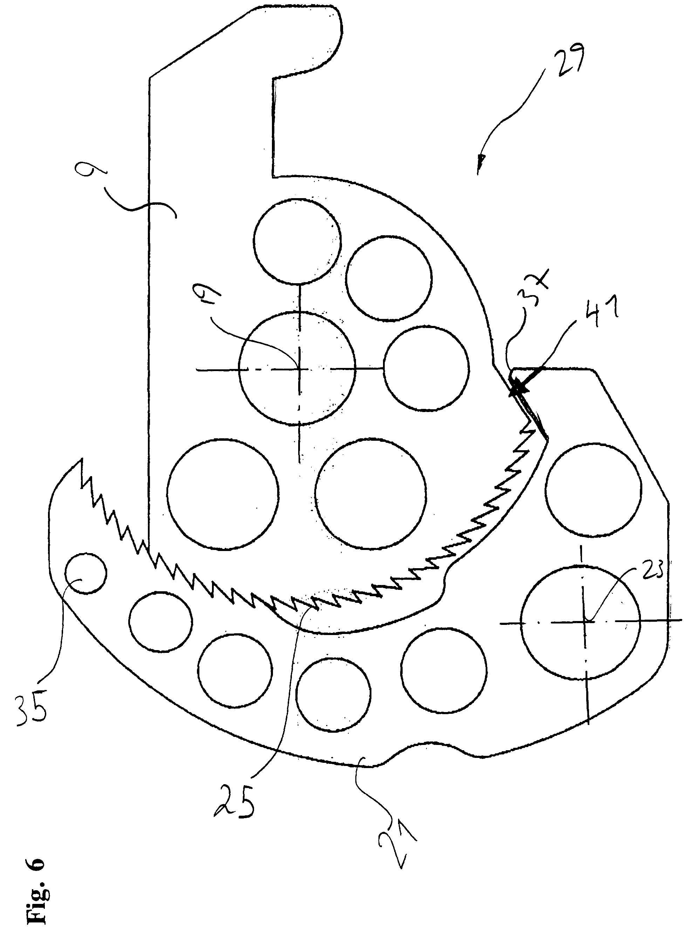

In Figur 6 ist das Gelenk 29 in einer der in Figur 2 dargestellten Position um etwa 100° bis 110° verdrehten Position dargestellt. Dies entspricht einer Abwinklung des Knies des Patienten, wenn dieser sich in der Hocke befindet. Damit die Abwinklung des Knies begrenzt wird und ein großes Drehmoment zwischen erstem Gelenkglied 9 und dem in Figur 6 nicht dargestellten zweiten Gelenkglied 15 übertragen werden kann, ist am ersten Gelenkglied 9 eine zweite Nocke 41 vorgesehen, die mit Erreichen des maximalen Beugungswinkels an der Vorderseite der Nase 37 der Zahnklinke 21 aufliegt und somit die Zahnklinke 21 in die Verzahnung 25 des ersten Gelenkglieds 9 dreht. Damit kann auch in dieser Extremsituation das gesamte zwischen erstem Gelenkglied 9 und dem in Figur 6 nicht dargestellten zweiten Gelenkglied 15 zu übertragende Drehmoment über das erfindungsgemäße Zahnrichtgesperre 31 übertragen werden.

Anschließend wird das erste Ausführungsbeispiel einer erfindungsgemäßen Auslöseeinrichtung 3 anhand der Fig. 1 erläutert. Die Zugfeder 26 zwischen Zahnklinke 21 und erstem Gelenkglied 9 zieht die Zahnklinke 21 immer in Richtung der Verzahnung 25. Um die Verriegelung zu lösen, ist es also erforderlich, dass die Seele 28 des Bowdenzugs 27 die Zahnklinke 21 aus der Verzahnung 25 herausdreht.

Wie aus Figur 1 ersichtlich, ist die Auslöseeinrichtung 3 unterhalb des nicht dargestellten Fußes des Patienten angeordnet. Sie kann vorzugsweise in einem speziell dafür umgearbeiteten Schuh des Patienten angeordnet werden. Alternativ können auch andere Auslösungeinrichtungen oddgl. eingesetzt werden, die die Be- oder Entlastungs des Beins erkennen.

Die Ausgleichseinrichtung 3 gemäß Figur 1 besteht im Wesentlichen aus einer Wippe 43, die in einer Wippenführung 45 um einen dritten Drehpunkt 47 drehbar gelagert ist. Die Hülle 49 des Bowdenzugs stützt sich mit einem Ende an der Wippenführung 45 ab und mit dem anderen Ende an einem Absatz 51, welcher in der Nähe der Zahnklinke 21 angeordnet ist und mit dem Unterteil 11 verbunden ist. Alternativ kann sich die Hülle 49 des Bowdenzugs 27 auch direkt am zweiten Gelenkglied 15 abstützen. Durch die Verwendung eines Bowdenzugs 27 in Verbindung mit einer Zugfeder 26 ergibt sich eine verbesserte Funktion und Sicherheit gegenüber einem starren Gestänge zwischen Auslöseeinrichtung 3 und Zahnklinke 21.

Die Seele 28 ist, wie bereits erwähnt, über eine Aufnahmebohrung 35 mit einem Ende an der Zahnklinke 21 befestigt und mit ihrem anderen Ende in der Wippe 43 eingehängt. Dazwischen kann eine nicht dargestellte Kupplung vorgesehen sein, die es dem Patienten erlaubt, die Auslöseeinrichtung 3 von der Gelenkorthese 1 abzunehmen. Wenn nun der nicht dargestellte Patient seinen Fuß, insbesondere seine Ferse belastet, dreht sich die Wippe 43 in die Wippenführung 21 in Richtung des Pfeils 53 und die Zugfeder 26 dreht die Zahnklinke 21 in die Verzahnung 25. Damit ist die Gelenkorthese 1 verriegelt.

Zwischen Wippe 43 und Wippenführung 45 ist ein nicht dargestelltes vorgespanntes Federelement vorhanden. Sobald die Ferse des Patienten entlastet wird, bewegt sich die Wippe 43 angetrieben von dem Federelement entgegen der Richtung des Pfeils 53 wieder aus der Wippenführung 45 heraus. Das Federelement kann als Blattfeder oder als Drahtfeder ausgeführt sein. Besonders vorteilhaft ist es, wenn zwischen Wippe 43 und Wippenführung 45 ein Moosgummistück eingepresst wird, welches einerseits die Funktion des Federelements übernimmt und andererseits das Eindringen von Schmutz oder Steinen in den Zwischenraum zwischen Wippe 43 und Wippenführung 45 verhindert. Dabei muß das Federelement die Federkraft der Zugfedern 26 in den Gelenken 29 und die Reibungswiderstände sicher überwinden.

In Figur 7 ist eine weitere Ausführungsform einer erfindungsgemäßen Auslöseeinrichtung 3 dargestellt. Um die Sichtbarkeit der wesentlichen konstruktiven Merkmale zu verbessern, ist die Auslöseeinrichtung 3 in Figur 7 auf dem Kopf dargestellt. Deshalb gehen die Hülle 49 und die Seele 28 des Bowdenzugs 27, anders als in Figur 1, nach unten weg. Die Wippenführung 45 weist zusätzlich eine Drehwinkelbegrenzung 55 auf, welche verhindert, dass die Wippe 43 sich zu weit aus der Wippenführung 45 herausdrehen kann. Zwischen der Wippenführung 45 mit einem U-förmigen Querschnitt und der als flacher Streifen ausgebildeten Wippe 43 ist eine Vorfußwippe 57 angeordnet, die ebenfalls um den dritten Drehpunkt 47 relativ zur Wippe 43 und zur Wippenführung 45 drehbar gelagert ist. Die Vorfußwippe hat ebenfalls ein U-förmiges Profil. Der Winkel, um den die Vorfußwippe 57 relativ zur Wippenführung 45 verdrehbar ist, ist in Figur 7 mit "β" bezeichnet. Bei diesem Ausführungsbeispiel ist die Hülle 49 des Bowdenzugs 27 nicht auf der Wippenführung 45 verankert, sondern auf der Vorfußwippe 57 ab. Wenn nun die Wippenführung 45 vom Ballen des in Figur 7 nicht dargestellten Patientenfußes in Richtung des Pfeils 59 auf den Boden gedrückt wird, dreht sich die Vorfußwippe 57 in (Figur 7 zeigt eine Auslöseeinrichtung 3 auf dem Kopf) die Wippenführung 45 hinein, so dass der Winkel β gleich Null wird. Das bedeutet, dass sich auf der anderen Seite des dritten Drehpunkts 47 der Winkel zwischen Wippe 43 und Vorfußwippe 57 verkleinert und somit die Seele 28 in die Hülle 49 in Richtung des Pfeils 61 hineinrutscht. Dadurch wird, wie aus Figur 1 ersichtlich, die Zahnklinke 21 in die Verzahnung 25 gedreht und somit die Gelenkorthese 1 verriegelt.

Zusätzlich oder alternativ ist auch möglich, die Gelenkorthese 1 von Hand zu verriegeln, so dass der Patient beispielsweise in unebenem Gelände immer einen sicheren Stand hat. Das Verriegeln kann dadurch geschehen, dass die Zahnklinke 21 über einen nicht dargestellten Hebel odgl. in die Verzahnung 25 gedreht und in dieser Position fixiert wird. In einer anderen Stellung des Hebels kann die Zahnklinke aus der Verzahnung 25 gedreht und in dieser Position fixiert werden, so dass die Gelenkorthese 1 entriegelt bleibt. In einer Neutralstellung des Hebels ist die Gelenkorthese auch verriegelt.

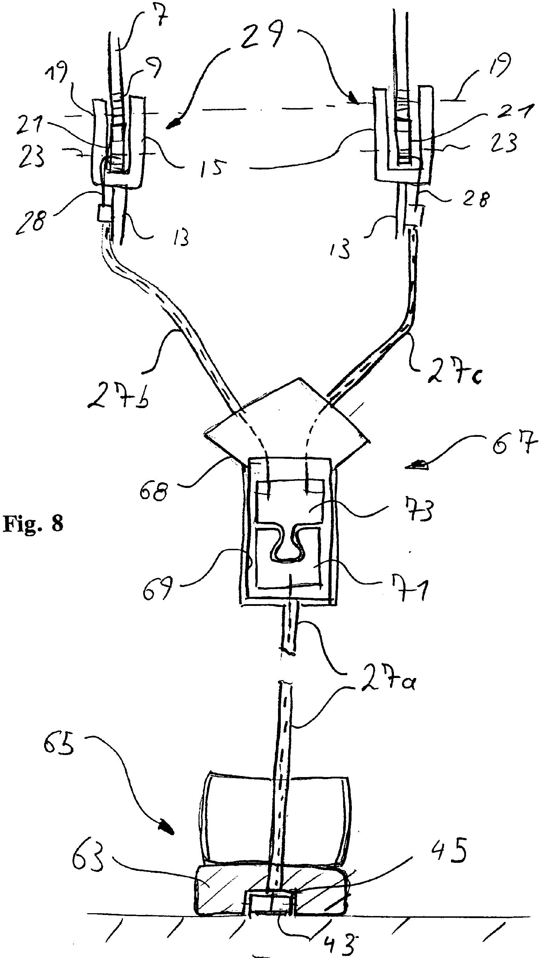

In Fig. 8 ist eine Gelenkorthese 1 mit einer Auslöseeinrichtung 3 von hinten dargestellt. In einen Absatz 63 eines geschnitten dargestellten Schuhs 65 ist eine Auslöseeinrichtung 3 mit Wippe 43 und Wippenführung 45 eingepasst.

Die Gelenkorthese 1 weist zu beiden Seiten des nicht dargestellten Patientenknies ein Gelenk 29 auf. In dieser Ansicht ist die U-förmige Ausgestaltung des zweiten Gelenkglieds 15 deutlich zu erkennen. Oberteil 5 und Unterteil 11 sind in Fig. 8 nicht dargestellt. In dem zweiten Gelenkglied 15 sind das erste Gelenkglied 9 und die Zahnklinke 21 gelagert.

Der Bowdenzug 27 teilt sich in einer Kupplung 67 auf, so dass die Ausgleichseinrichtung 3 beide Gelenke 29 synchron betätigen kann. In einer Aussparung 69 sind ein erstes Kupplungsstück 71 und ein zweites Kupplungsstück 73 formschlüssig miteinander verbunden. Die Seele des Bowdenzuabschnitts 27a ist mit dem ersten Kupplungsstück 71 verbunden. Die Seelen der Bowdenzuabschnitte 27b und 27c sind mit dem zweiten Kupplungsstück 73 verbunden. dadurch, dass sich die Bowdenzubaschnitte 27a, 27b und 27c an dem Kupplungsgehäuse 67 abstützen wird die Relativbewegung zwischen Wippe 43 und Wippenführung 45 auf beide Zahnklinken 21 übertragen. Wenn der Schuh 65 von der Gelenkorthese 1 abgenommen werden soll, muß der Patient lediglich den Formschluß zwischen erstem und zweitem Kupplungsstück 71 und 73 aufheben. Anschließend kann der Patient den Schuh 65 von der Gelenkorthese 1 abnehmen. Dabei wird z. B. der Bowdenzugabschnitt 27a von der Kupplung 67 getrennt.

Durch einen nicht dargestellten Deckel kann die Ausnehmung 69 des Kupplungsgehäuses 68 verschlossen und somit ein unbeabsichtigtes Lösen von erstem und zweitem Kupplungsstück 71 und 73 sowie des distalen Bowdenzugs verhindert werden. Mittels nicht dargestellter Stellschrauben im Bereich der Bowdenzugabschnitte 27a, 27b und 27c kann kann die Gelenkorthese 1 eingestellt und Fertigungstoleranzen ausgeglichen werden.

Alle in der Beschreibung, den nachfolgenden Ansprüchen und der Zeichnung dargestellten Merkmale können sowohl einzeln als auch in beliebiger Kombination miteinander erfindungswesentlich sein.

## Patentansprüche

1. Gelenkorthese zur Unterstützung eines Gelenks, insbesondere eines Kniegelenks, mit einem ersten Gelenkglied (9), mit einem zweiten Gelenkglied, mit einem Zahnrichtgesperre (31), wobei das erste Gelenkglied (9) und das zweite Gelenkglied (15) mit einem Gelenkbolzen (17) drehbar miteinander verbunden sind, wobei das erste Gelenkglied (9) ein Sperrstück () mit einer Verzahnung (25) aufweist und wobei an dem zweiten Gelenkglied (15) eine mit der Verzahnung (25) zusammenwirkende Zahnklinke (21) drehbar angeordnet ist, **dadurch gekennzeichnet, dass** das Zahnrichtgesperre (31) beim Erreichen eines ersten Drehwinkelanschlags entriegelt wird, und dass das Zahnrichtgesperre (31) beim Erreichen eines zweiten Drehwinkelanschlags verriegelt wird.

2. Gelenkorthese nach Anspruch 1, **dadurch gekennzeichnet, dass** das erste Gelenkglied (9) einen ersten Nocken (39) aufweist, der beim Erreichen des ersten Drehwinkelanschlags die Zahnklinke (21) aus der Verzahnung (25) hebt.

3. Gelenkorthese nach Anspruch 1 oder 2, **dadurch gekennzeichnet, dass** der Eingriffswinkel α der Verzahnung (25) in Belastungsrichtung negativ ist und vorzugsweise - 10° bis -30°, besonders bevorzugt -20°, beträgt.

4. Gelenkorthese nach einem der vorhergehenden Ansprüche, **dadurch gekennzeichnet, dass** das zweite Gelenkglied (15) die Verzahnung (25) beidseitig umgibt, und dass der Gelenkbolzen (17) zweifach in dem zweiten Gelenkstück (15) gelagert ist.

5. Gelenkorthese nach einem der vorhergehenden Ansprüche, **dadurch gekennzeichnet, dass** zwischen Gelenkbolzen (17) sowie erstem und zweitem Gelenkglied (15) eine Büchse, insbesondere aus Bronze mit Teflon-Beschichtung vorgesehen ist.

6. Gelenkorthese nach einem der vorhergehenden Ansprüche, **dadurch gekennzeichnet, dass** zu beiden Seiten des zu unterstützenden Gelenks je ein Gelenk (29) mit einem ersten Gelenkglied (9), einem zweiten Gelenkglied (15) und einem Zahnrichtgesperre (31) angeordnet ist, und dass die Drehachsen () der Gelenke (29) koaxial verlaufen.

7. Gelenkorthese nach einem der vorhergehenden Ansprüche, **dadurch gekennzeichnet**, und dass das erste Gelenkglied (9) einen zweiten Nocken (41) aufweist, der beim Erreichen eines zweiten Drehwinkelanschlags () die Zahnklinke (21) in die Verzahnung (25) einrastet.

8. Gelenkorthese nach einem der vorhergehenden Ansprüche, **dadurch gekennzeichnet, dass** das Zahnrichtgesperre (31) von einer Auslöseeinrichtung (3) entriegelbar oder verriegelbar ist.

9. Gelenkorthese nach einem der vorhergehenden Ansprüche, **dadurch gekennzeichnet, dass** die Zahnklinke (21) federbelastet in die Verzahnung (25) bewegt wird.

10. Gelenkorthese nach einem der vorhergehenden Ansprüche, **dadurch gekennzeichnet, dass** die Zahnklinke (21) von der Auslöseeinrichtung (3) aus der Verzahnung (25) drehbar ist.

11. Gelenkorthese nach einem der vorhergehenden Ansprüche, **dadurch gekennzeichnet, dass** die Auslöseeinrichtung (3) in oder an einem Schuh angeordnet ist, und dass die Auslöseeinrichtung (3) eine drehbar in einer Wippenführung (45) gelagerte Wippe (43) aufweist, dass sich an der Wippenführung (45) und an dem zweiten Gelenkglied (15) eine Bowdenzughülle mindestens mittelbar abstützt, und dass Wippe und Zahnklinke (21) durch die Seele (28) des Bowdenzugs (27) mindestens mittelbar gekoppelt sind.

12. Gelenkorthese nach Anspruch 11, **dadurch gekennzeichnet, dass** an der Wippenführung (45) eine Drehwinkelbegrenzung (55) für die Wippe (43) vorhanden ist.

13. Gelenkorthese nach Anspruch 11 oder 12, **dadurch gekennzeichnet, dass** zwischen Wippenführung (45) und Wippe (43) eine Vorfusswippe (57) angeordnet ist, das die Vorfußwippe (57) koaxial zum Drehpunkt (19) der Wippe (43) gelagert ist, dass die Wippenführung (45) und die Vorfusswippe (47) über den Drehpunkt (19) der Wippe (43) hinaus verlängert sind,

14. Gelenkorthese nach einem der Ansprüche 11 bis 13, **dadurch gekennzeichnet, dass** ein Federelement () die Wippe (43) so zur Wippenführung (45) und/oder zur Vorfußwippe (57) verdreht, dass die Zahnklinke (21) durch das Federelement () aus der Verzahnung (25) gedreht wird.

15. Gelenkorthese nach Anspruch 14, **dadurch gekennzeichnet, dass** das Federelement () ein zwischen Wippe (43) und Wippenführung (45)/ oder Vorfußwippe (57) angeordnetes Mossgummi () oder eine Druckfeder ist.

16. Gelenkorthese nach Anspruch 14, **dadurch gekennzeichnet, dass** das Federelement () eine Blattfeder () ist.

17. Gelenkorthese nach einem der Ansprüche 8 bis 16, **dadurch gekennzeichnet, dass** zwischen Auslöseeinrichtung (3) und Gelenk (29) eine Einstelleinrichtung, insbesondere eine Stellschraube () vorgesehen ist.

18. Gelenkorthese nach einem der Ansprüche 11 bis 17, **dadurch gekennzeichnet, dass** die Wippe (43) beim Stehen durch das Gewicht des Patienten auch gegen die Federkraft eines evtl. vorhandenen Federelelements () so zur Wippenführung (45) verdreht wird, dass die Zahnklinke (21) in die Verzahnung (25) einrastet.

19. Gelenkorthese nach einem der Ansprüche 11 bis 18, **dadurch gekennzeichnet, dass** durch einen Druck auf den vor dem Drehpunkt (19) der Wippe (43) gelegenen Teil der Vorfußwippe (57) auch gegen die Federkraft eines evtl. vorhandenen Federelelements () so zur Wippenführung (45) verdreht wird, dass die Zahnklinke () in die Verzahnung (25) einrastet.

20. Gelenkorthese nach einem der Ansprüche 8 bis 19, **dadurch gekennzeichnet, dass** zwischen Auslöseeinrichtung (3) und Gelenk (29) eine Kupplung (67) vorgesehen ist.

21. Gelenkorthese nach Anspruch 20, **dadurch gekennzeichnet, dass** die Kupplung (67) ein erstes mit dem zweiten Gelenkglied (15) mindestens mittelbar verbundenens erstes Kupplungsstück (71) aufweist, dass die Kupplung (67) ein zweites mit der Auslöseeinrichtung (3) mindestens mittelbar verbundenes zweites Kupplungsstück (73) aufweist, und dass erstes und zweites Kupplungsstück (71, 73) formschlüssig miteinander verbindbar sind.

22. Gelenkorthese nach einem der vorhergehenden Ansprüche, **dadurch gekennzeichnet, dass** die Zahnklinke (21) von Hand in die Verzahnung (25) oder aus der Verzahnung (25) gedreht werden und in beiden Positionen sowie in einer Neutralstellung feststellbar ist.
